# EUROPÄISCHE PATENTSCHRIFT

(11) **EP 0 892 924 B1**
(45) Veröffentlichungstag und Bekanntmachung des Hinweises auf die Patenterteilung: **17.03.2004**
(21) Anmeldenummer: 97922829.3
(22) Anmeldetag: 09.04.1997
(51) Int. Cl.: G01N 33/34, G01N 21/35, G01N 21/86

(54) **VERFAHREN ZUR BESTIMMUNG DER MECHANISCHEN EIGENSCHAFTEN VON PAPIER UND ZUGEHÖRIGE ANORDNUNG**
PROCESS AND DEVICE FOR DETERMINING THE MECHANICAL PROPERTIES OF PAPER
PROCEDE ET DISPOSITIF DE DETERMINATION DES PROPRIETES MECANIQUES DU PAPIER

(30) Priorität: 09.04.1996 DE 19613986
(43) Veröffentlichungstag der Anmeldung: 27.01.1999
(73) Patentinhaber: SIEMENS AKTIENGESELLSCHAFT, 80333 München (DE)
(72) Erfinder: FURUMOTO, Herbert, D-91052 Erlangen (DE); LAMPE, Uwe, D-85435 Erding (DE); ROTH, Christoph, D-81739 München (DE)
(86) Internationale Anmeldenummer: PCT/DE1997/000724
(87) Internationale Veröffentlichungsnummer: WO 1997/038305

(56) Entgegenhaltungen:
- EP-A- 0 311 518
- EP-A- 0 464 958
- WO-A-95/31709
- GB-A- 2 147 413
- PAPIER, DAS, Bd. 45, Nr. 2, 1991, DARMSTADT, DE, Seiten 45-51, XP002039457 D. FENGEL ET AL.: in der Anmeldung erwähnt

## Beschreibung

Verfahren zur Bestimmung der mechanischen Eigenschaften von Papier und zugehörige Anordnung

Die Erfindung bezieht sich auf ein Verfahren zur Bestimmung der mechanischen Eigenschaften von Papier, insbesondere zur Messung der Faserbindungen im Papier. Daneben bezieht sich die Erfindung auch auf die zugehörige Anordnung zur Durchführung des Verfahrens, mit einem Spektrometer aus Strahlquelle, Optik und einem Detektor.

Bei der Produktion von Papier und/oder Karton ist eine ständige Überprüfung der mechanischen Eigenschaften für die Qualitätssicherung notwendig. Diese Eigenschaften hängen von der Art und Anzahl der Faserbindungen ab. Angestrebt wird dazu eine direkte Messung der Faserbindungen im Papier während der Produktion, was eine Online-Regelung im Herstellungsprozeß erlauben würde.

Eine direkte Messung der Faserbindungen ist bisher nicht möglich. Es ist kein Sensor bekannt, mit dem direkt gemessen werden kann, wie gut und wie fest die einzelnen Zellulosefasern im Papier und/oder Karton aneinandergebunden sind. Vorgeschlagen wurden jedoch bereits eine Reihe von Meßmethoden zur Untersuchung der Festigkeit von Papier, welche unmittelbar oder mittelbar mit den Faserbindungen in der Zellulose zusammenhängen. Die Messungen werden jedoch selbst durch die Festigkeit der einzelnen Fasern beeinflußt, so daß keine direkte Korrelation zum gewünschten Meßergebnis besteht.

Bisher werden in der Papierfabrik laufend Laboruntersuchungen beispielsweise an der Papiermaschine zur Festigkeit des Papiers durchgeführt, um einen bestimmten Qualitätsstandard zu sichern. Dazu werden der laufenden Papierbahn Proben entnommen und im Labor auf Eigenschaften, wie z.B. Reißlänge, Durchreißfestigkeit, Gasdruck od. dgl., untersucht. Solche Messungen sind im allgemeinen zeitaufwendig und erfordern qualifiziertes Personal.

Es wurde bereits ein Online-Sensor vorgeschlagen, der an der laufenden Papierbahn die Steigung der Kraft-Dehnungs-Kurve über die Ausbreitungsgeschwindigkeit von Ultraschall im Papier mißt. Der Sensor besteht aus einer Ultraschallquelle und zwei Detektoren in unterschiedlichem Abstand. Aus der Differenz der Laufzeiten des Ultraschallpulses an den beiden Detektoren kann die Papierfestigkeit bestimmt werden. Dieser Wert ist im wesentlichen durch die Steigung der Kurve im Kraft-Dehnungs-Diagramm bestimmt.

In den Zeitschriften-Veröffentlichungen "Das Papier" (1991), S.45 - 51 und "Das Papier" (1993), S.695 - 702 wird bereits über Möglichkeiten und Grenzen der FTIR-Spektroskopie bei der Charakterisierung von Cellulose berichtet. Dabei werden insbesondere spektroskopische und elektronenmikroskopische Untersuchungen zur Feinstruktur der Cellulose miteinander verglichen. Praktische Folgerungen können daraus nicht gezogen werden.

Weiterhin werden in Tappi-Journal (1992),P. 147 -149 Aussagen darüber gemacht, daß mittels optischer Infrarotmessungen der Ligningehalt einer Zellstoff-Pulpe, d.h. dem Zellstoff in breiiger Konsistenz,gemessen werden kann.

Aus EP-A-0 464 958 ist ein Verfahren zur Bestimmung der Festigkeit von Papier bekannt, wobei u.a. ein Infrarot Fenchtigkeitssensor verwendet wird.

Aus WO-A-95 31709 ist ein Verfahren zur Bestimmung der Naßfestigkeit von Papier bekannt, wobei ein Infrarotspektrum ermittelt und ausgewerkt wird.

Aufgabe der Erfindung ist es daher, ein Verfahren anzugeben und die zugehörige Anordnung zu schaffen, die zur Messung der Faserbindungen im Papier eingesetzt werden können.

Die Aufgabe ist erfindungsgemäß durch die Anwendung von Infrarot-Spektroskopie gelöst, wobei das Vorliegen von an der Faseroberfläche miteinander verbundenen Hydroxyl- und/oder Carboxylgruppen optisch erfaßt und als Maß für die mechanischen Eigenschaften des Papiers ausgewertet werden. Bei der zugehörigen Anordnung ist eine Auswerteeinheit vorhanden, bei der eine Basislinienkorrektur der Spektren durchgeführt wird und anschließend anhand der Banden im Spektrum die mechanischen Eigenschaften, insbesondere das Vorliegen von Faserbindungen, bestimmt werden.

Die Erfindung stellt also erstmalig einen praxigerechten Sensor zur Verfügung, der mit Hilfe der Infrarot-Spektroskopie (IR) direkt die Faserqualität im Papier zu erfassen vermag. Die IR-Spektroskopie ist zwar ein an sich bekanntes Verfahren zur chemischen Charakterisierung von Stoffen und wurde - wie eingangs erwähnt - bereits auch zur Untersuchung von Cellulose vorgeschlagen. Nunmehr kann aber mit der IR-Spektroskopie unmittelbar die Faserqualität von Papier und/oder Karton bestimmt werden.

Im Rahmen der Erfindung wurde die nichttriviale Erkenntnis bestätigt, daß die mechanischen Eigenschaften des Papiers hauptsächlich durch die Qualität der Zellulosefasern bestimmt werden. Die Faserqualität ist ein Maß für ihre Eigenschaft, mit anderen Fasern durch feste Bindungen einen stabilen Verbund zu bilden und somit die mechanischen Eigenschaften des Papiers gewährleistet. Die Festigkeit dieser Interfaserverbindungen wird nach der vorherrschenden Theorie durch die Konzentration der Hydroxylgruppen und/oder Carboxylgruppen an der Faseroberfläche bestimmt, die über Wasserstoffbrückenbindungen aneinandergekettet sind. Durch unerwünschte Störstoffe können die Fasern an einer Bindung gehindert werden. In diesem Fall sind freie OH-Gruppen vorhanden, die nicht über Bindungen abgesättigt werden. Je höher die Konzentration der gesättigten OH-Gruppen ist, desto höher ist die Festigkeit des Papiers.

Weitere Eigenschaften und Vorteile der Erfindung ergeben sich aus der nachfolgenden Figurenbeschreibung von Ausführungsbeispielen anhand der Zeichnung in Verbindung mit weiteren Unteransprüchen. Es zeigen
- Figur 1: ein Infrarotspektrum von altpapierhaltigem Papier,
- Figur 2: das Infrarotspektrum von Figur 1 nach einer Basislinienkorrektur,
- Figur 3: ein Infrarotspektrum von holzfreiem Papier und
- Figur 4: ein Blockschaltbild mit zugehörigen Auswerteeinhei ten.

Die Infrarot-Spektroskopie ist ein Standardverfahren in der chemischen Industrie zur Charakterisierung von Stoffen. Speziell zur Messung an Papier ist unter Berücksichtigung der eingangs genannten Ausführungen zum Aufbau der Cellulosefasern folgendes zu berücksichtigen:

Die Resonanzschwingungsfrequenz von freien OH-Gruppen liegt im Bereich von ca. 3700 cm⁻¹. Werden zwei OH-Gruppen über Wasserstoffbrückenbindungen verbunden, sind die OH-Gruppen in ihrer Schwingung behindert und die Resonanzfrequenz verschiebt sich in den Bereich von ca. 3200 cm⁻¹ bis 3400 cm⁻¹. Es läßt sich also durch die spektroskopische Messung unmittelbar feststellen, ob OH-Gruppen frei oder gebunden sind. Durch Auswertung des Spektrums von Papier läßt sich darüber hinaus quantitativ ermitteln, wieviele OH-Gruppen zu den Faserbindungen beitragen und wieviele OH-Gruppen frei sind. Je mehr OH-Gruppen aneinandergebunden sind, desto größer ist die Festigkeit des Papiers.

Für die praktische Anwendung ist ein Infrarotspektrometer zu verwenden, das dem Bereich von 3500 cm⁻¹ bis 3800 cm⁻¹ abdeckt. Ein übliches Spektrometer, das hier nicht im einzelnen dargestellt ist, besteht aus einer Strahlquelle lichtführender Optik und einem Detektor. Gegebenenfalls kann auch ein sogenanntes Fouriertransform-Spektrometer verwendet werden, mit dem die Signale unmittelbar in bearbeiteter Form ausgegeben werden.

Eine Papierprobe wird an geeigneter Stelle in das Spektrometer eingebracht und entweder vom Infrarotstrahl (IR) durchleuchtet oder es wird die diffuse Reflexion des IR-Strahlung gemessen. Wird die Probe durchleuchtet, was nur bei Papieren, nicht aber bei Karton realisierbar ist, erfolgt eine Transmissionsmessung. Ebensogut ist auch eine Reflexion möglich. Bei Messung der diffusen Reflexion wird dagegen das direkt reflektierte Licht ausgeblendet und das diffus reflektierte Licht mit Hohlspiegeln fokussiert und auf den Detektor gelenkt. Die spektrale Auflösung des Spektrometers muß für die bestimmungsgemäße Anwendung besser als 10 cm⁻¹ sein.

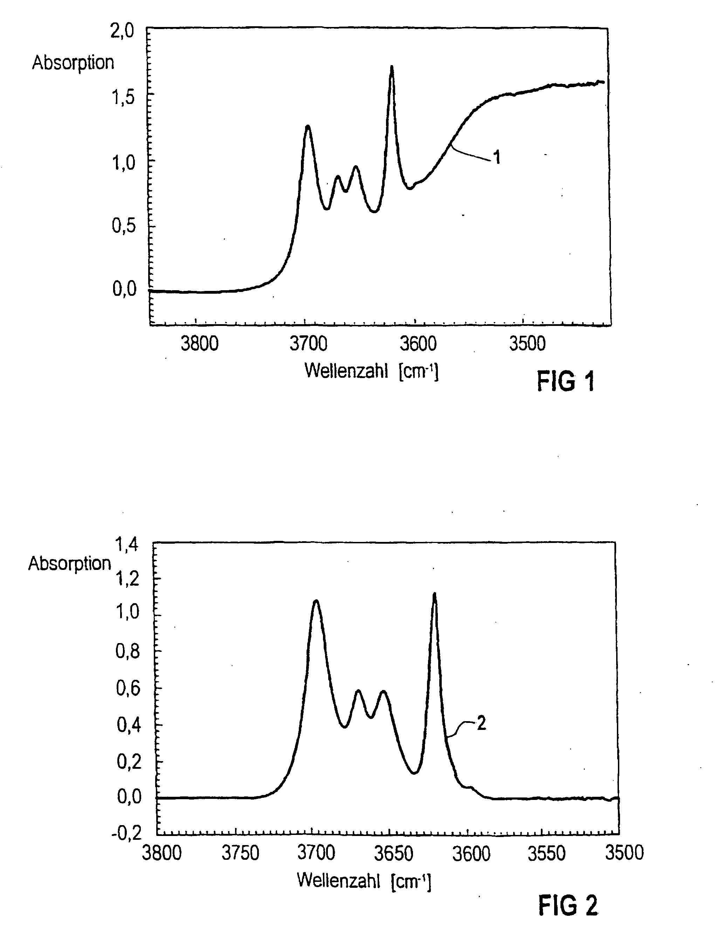

In Figur 1 ist ein IR-Spektrum 1 im Bereich von ca. 3500 cm⁻¹ bis 3800 cm⁻¹ dargestellt, das von Papier mit hohem Altpapieranteil erhalten wurde. An einem solchen Rohspektrum mit als Banden interpretierbarer Struktur wird zunächst mit einer Recheneinheit eine Basislinienkorrektur durchgeführt, wodurch ein korrigiertes Spektrum 2 gemäß Figur 2 erhalten wird. Letzteres Spektrum 2 ist durch signifikante Banden gekennzeichnet. Ein solches Spektrum kann zwecks weiterer Auswertung durch schnelle Fourier-Transformation(FFT) bearbeitet werden.

Letztere Banden im Spektrum kennzeichnen die Schwingungen der freien OH-Gruppen, d.h. derjenigen OH-Gruppen, die keine Bindungen eingegangen sind. Diese Banden treten nur auf, wenn nicht alle OH-Gruppen an den Bindungen beteiligt sind, wenn also die Fasern nicht optimal miteinander verbunden sind.

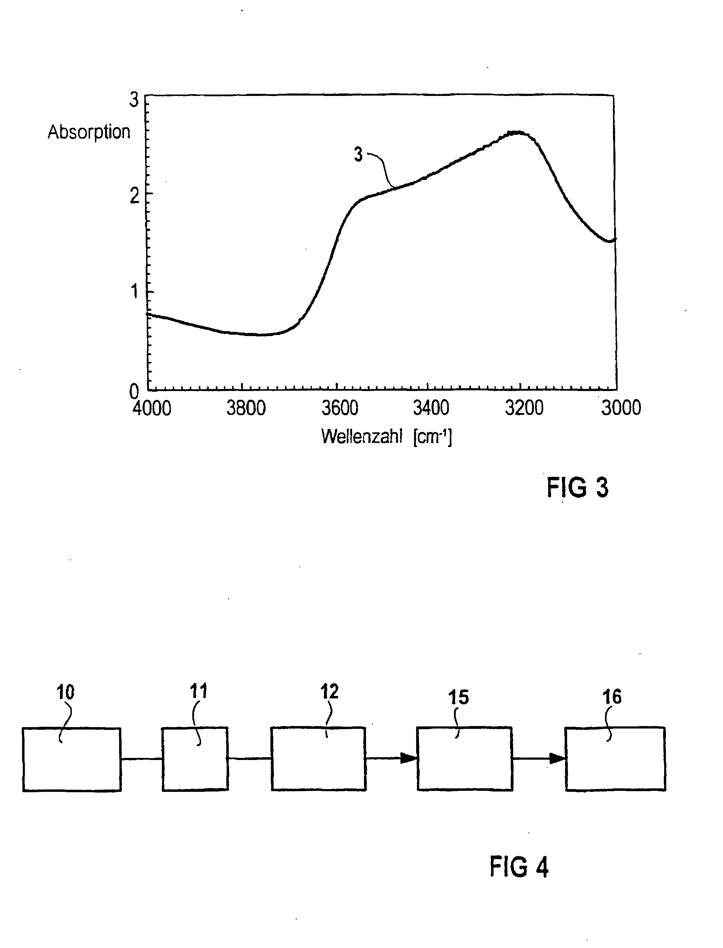

Figur 3 zeigt ein Spektrum 3 von hochwertigem holzfreiem Papier, ohne daß hier bereits eine Basislinienkorrektur durchgeführt wurde. Man erkennt aber trotzdem deutlich, daß keine der oben abgebildeten Banden zu erkennen sind. Dies bedeutet, daß die Fasern optimal miteinander verbunden sind.

Im zugehörigen Blockschaltbild gemäß FIG 4 beinhaltet eine Einheit 10 ein IR-Spektrum, das am Papier gemessen wurde, beispielsweise das Spektrum 1. Mit 11 ist eine Einheit zur schnellen Fourriertransformation (FFT) dargestellt, der eine Einheit 12 zur mathematischen Vorverarbeitung nachfolgt. In letzterer Einheit 12 erfolgt ein Glätten der Signale und die bereits erwähnte Basislinienkorrektur. Nach einem Dekonvulieren der Signale ist die Ermittlung von Linienbreiten, die Ermittlung von Intensitäten sowie eine Komponentenanalyse möglich.

In FIG 4 beinhaltet Einheit 15 ein mathematisches Modell "Papierqualität". Dieses Modell korreliert Ergebnisse der Spektralanalyse und zugehöriger Auswertung mit den Kriterien von Papierqualität od. dgl.. Von der Einheit 15 wird die Einheit 16 angesteuert, die zur Ermittlung von Maßnahmen zur Sicherung der Papierqualität dient.

Bei der Infrarotmessung kann der Sensor direkt an der laufenden Papierbahn angebracht sein. Dies ermöglicht eine schnelle Online-Kontrolle der Papierqualität. Möglicherweise kann der Sensor auch in einen Meßrahmen eingebaut werden, der sich transvernierend über die Papierbahn bewegt, wie er beispielsweise vorteilhaft für Messungen wie Flächendichte und/oder Feuchtigkeitsgehalt quer zur Papierbahn vorgeschlagen wurde. Für eine Transmissionsmessung kann die Strahlquelle im oberen Teil des Meßrahmens und der Detektor im unteren Teil angebracht werden. Bei Messungen der diffusen Reflexion sind dagegen sowohl Strahlquelle als auch Detektor beide im oberen Teil oder aber im unteren Teil des Meßrahmens.

Abgesehen von den beschriebenen Online-Messung würde auch ein Offline-Sensor noch ein en erheblichen Zeitvorteil gegenüber Labormessungen liefern. In diesem Fall wird aus der Papierbahn eine Probe entnommen und im Labor in einen IR-Spektrometer vermessen. Solche Messungen können innerhalb weniger Minuten durchgeführt werden.

## Patentansprüche

1. Verfahren zur Bestimmung der mechanischen Eigenschaften von Papier, insbesondere zur Messung der Faserbindungen im Papier, **gekennzeichnet durch** die Anwendung von Infrarot-Spektroskopie, wobei das Vorliegen von an der Faseroberfläche miteinander verbundenen Hydroxyl- und/oder Carboxylgruppen optisch erfaßt und als Maß für die mechanischen Eigenschaften des Papiers ausgewertet werden.

2. Verfahren nach Anspruch 1, **dadurch gekennzeichnet, daß** die Festigkeit des Papiers durch die Faserbindungen bestimmt und damit proportional der Konzentration der gesättigten OH-Gruppen ist.

3. Verfahren nach Anspruch 1, **dadurch gekennzeichnet, daß** in Transmission gemessen wird.

4. Verfahren nach Anspruch 1, **dadurch gekennzeichnet, daß** in Reflexion gemessen wird, wobei das direkt reflektierte Licht ausgeblendet und das diffus reflektierte Licht zur Auswertung verwendet wird.

5. Verfahren nach einem der vorhergehenden Ansprüche, **dadurch gekennzeichnet, daß** Online an einer laufenden Papierbahn gemessen wird.

6. Verfahren nach einem der Ansprüche 3 und 5, **dadurch gekennzeichnet, daß** traversierend über die Papierbahn gemessen wird.

7. Verfahren nach Anspruch 6, **dadurch gekennzeichnet, daß** die Auswertung online erfolgt und das Ergebnis bei der Prozeßführung berücksichtigt wird.

8. Verfahren nach einem der vorhergehenden Ansprüche, **dadurch gekennzeichnet, dass** diskontinuierlich nach einer Probennahme gemessen wird.

9. Verfahren nach einem der vorhergehenden Ansprüche, **dadurch gekennzeichnet, dass** zur Auswertung eine Fourier-Transformation der Signale durchgeführt wird.

10. Anordnung zur Durchführung des Verfahrens nach Anspruch 1 oder einem der Ansprüche 2 bis 9, mit einem Spektrometer aus Strahlquelle, Optik und einem Detektor, **gekennzeichnet durch** Auswerteeinheiten (11 - 16), mit denen insbesondere eine Basislinienkorrektur der Spektren durchgeführt und bei der anhand der Banden im Spektrum die mechanischen Eigenschaften, insbesondere das Vorliegen von Faserbindungen, bestimmt wird.

11. Anordnung nach Anspruch 10, **dadurch gekennzeichnet, dass** eine Einheit (12) zur mathematischen Vorverarbeitung, insbesondere zur Basislinienkorrektur der Spektren, vorhanden ist.

12. Anordnung nach Anspruch 10, **dadurch gekennzeichnet, dass** eine Einheit (15) vorhanden ist, die ein Modell für die Papierqualität beinhaltet und die Ergebnisse der Spektralanalyse mit Größen für die Papierqualität korreliert.

## Claims

1. Method of determining the mechanical properties of paper, in particular of measuring the fibre bonds in the paper, **characterized by** the use of infrared spectroscopy, wherein the presence of hydroxyl and/or carboxyl groups joined to one another at the fibre surface is optically detected and evaluated as a measure of the mechanical properties of the paper.

2. Method according to claim 1, **characterized in that** the strength of the paper is determined by the fibre bonds and is therefore proportional to the concentration of the saturated OH groups.

3. Method according to claim 1, **characterized in that** measurement is effected in transmission.

4. Method according to claim 1, **characterized in that** measurement is effected in reflection, wherein the directly reflected light is screened out and the diffusely reflected light is used for evaluation.

5. Method according to one of the preceding claims, **characterized in that** measurement is effected on-line at a moving paper web.

6. Method according to one of claims 3 and 5, **characterized in that** measurement is effected with traversing over the paper web.

7. Method according to claim 6, **characterized in that** the evaluation is effected on-line and the result is taken into account for the process management.

8. Method according to one of the preceding claims, **characterized in that** measurement is effected discontinuously after a sample is taken.

9. Method according to one of the preceding claims, **characterized in that** for evaluation purposes a Fourier transformation of the signals is effected.

10. Arrangement for effecting the method according to claim 1 or one of claims 2 to 9, comprising a spectrometer composed of beam source, optical system and a detector, **characterized by** evaluation units (11-16), by means of which in particular a baseline correction of the spectra is effected, and wherein from the bands in the spectrum the mechanical properties are determined, in particular the presence of fibre bonds.

11. Arrangement according to claim 10, **characterized in that** a unit (12) is provided for mathematical preprocessing, in particular for baseline correction of the spectra.

12. Arrangement according to claim 10, **characterized in that** a unit (15) is provided, which contains a model for the paper quality and correlates the results of the spectral analysis with variables for the paper quality.

## Revendications

1. Procédé de détermination des propriétés mécaniques du papier, notamment de mesure des liens interfibreux du papier, **caractérisé par** l'utilisation de la spectroscopie infrarouge, la présence de groupes hydroxyle et/ou carboxyle, reliés entre eux à la surface des fibres, étant détectée optiquement et étant exploitée comme mesure des propriétés mécaniques du papier.

2. Procédé suivant la revendication 1, **caractérisé en ce que** la solidité du papier est déterminée par les liens interfibreux et est ainsi proportionnelle à la concentration des groupes OH saturés.

3. Procédé suivant la revendication 1, **caractérisé en ce que** l'on effectue la mesure en transmission.

4. Procédé suivant la revendication 1, **caractérisé en ce que** l'on effectue la mesure en réflexion, la lumière réfléchie directement étant supprimée et la lumière réfléchie de manière diffuse étant utilisée pour l'exploitation.

5. Procédé suivant l'une des revendications précédentes, **caractérisé en ce que** l'on effectue une mesure en ligne sur une bande de papier mobile.

6. Procédé suivant l'une des revendications 3 et 5, **caractérisé en ce que** l'on effectue la mesure en travers sur la bande de papier.

7. Procédé suivant la revendication 6, **caractérisé** en ce l'on effectue l'exploitation en ligne et en ce que l'on tient compte du résultat pour la conduite des opérations.

8. Procédé suivant l'une des revendications précédentes, **caractérisé en ce que** l'on effectue la mesure en discontinu après une prise d'échantillon.

9. Procédé suivant l'une des revendications précédentes, **caractérisé en ce que** pour l'exploitation, on effectue une transformation de Fourier des signaux.

10. Dispositif de mise en oeuvre du procédé suivant la revendication 1 ou l'une des revendications 2 à 9, comprenant un spectromètre constitué d'une source de rayonnement, d'une optique et d'un détecteur, **caractérisé par** des unités (11 à 16) d'exploitation par lesquelles on effectue notamment une correction de ligne de base des spectres et dans laquelle on détermine, au moyen des bandes du spectre, les propriétés mécaniques, notamment la présence de liens interfibreux.

11. Dispositif suivant la revendication 10, **caractérisé en ce qu'**il est prévu une unité (12) de prétraitement mathématique, notamment de correction de ligne de base des spectres.

12. Dispositif suivant la revendication 10, **caractérisé en ce qu'**il est prévu une unité (15) qui comporte un modèle pour la qualité du papier et qui correlle les résultats de l'analyse des spectres à des grandeurs pour la qualité du papier.
